# FASCICULE DE BREVET EUROPEEN

(11) **EP 1 089 994 B1**
(45) Date de publication et mention de la délivrance du brevet: **08.09.2004**
(21) Numéro de dépôt: 99923712.6
(22) Date de dépôt: 10.06.1999
(51) Int. Cl.: C07D 403/10, A61K 31/415

(54) **NOUVELLE FORME DE L'IRBESARTAN, PROCEDES POUR OBTENIR LADITE FORME ET COMPOSITIONS PHARMACEUTIQUES EN CONTENANT**
NEUE FORM VOM IRBESARTAN, VERFAHREN UM DIESE FORM ZU ERHALTEN UND SIE ENTHALTENDE PHARMAZEUTISCHE ZUSAMMENSETZUNGEN
NOVEL FORM OF IRBESARTAN, METHODS FOR OBTAINING SAID FORM AND PHARMACEUTICAL COMPOSITIONS CONTAINING SAME

(30) Priorité: 24.06.1998 FR 9808037
(43) Date de publication de la demande: 11.04.2001
(73) Titulaire: SANOFI-SYNTHELABO, 75013 Paris (FR)
(72) Inventeur: FRANC, Bruno, F-30650 Saze (FR); HOFF, Christian, F-30126 Saint Laurent des Arbres (FR); KIANG, San, Madison, NJ 07940 (US); LINDRUD, Mark, D., Madison, NJ 07940 (US); MONNIER, Olivier, F-34560 Villeveyrac (FR); WEI, Chenkou, Princeton Junction, NJ 08688 (US)
(74) Mandataire: Thouret-Lemaitre, Elisabeth
(86) Numéro de dépôt international: PCT/FR1999/001372
(87) Numéro de publication internationale: WO 1999/067236

(56) Documents cités:
- EP-A- 0 708 103
- WO-A-91/14679
- M. BAUER ET AL.: "NMR study of desmotropy in Irbesartan, a tetrazole-containing pharmaceutical compound" JOURNAL OF THE CHEMICAL SOCIETY, PERKIN TRANS. 2, no. 2, mars 1998 (1998-03), pages 475-481, XP002112787 CHEMICAL SOCIETY. LETCHWORTH., GB
- CHEMICAL ABSTRACTS, vol. 129, no. 6, 10 août 1998 (1998-08-10) Columbus, Ohio, US; abstract no. 74251, BOCSKEI Z. ET AL.: "Irbesartan crystal form B" XP002112788 & ACTA CRYSTALLOGR., SECT. C: CRYST. STRUCT. COMMUN., vol. 54, no. 6, 1998, pages 808-810,

## Description

La présente invention a pour objet un nouvel habitus cristallin du 2-n-butyl-4-spirocyclopentane-1-[(2'-(tétrazol-5-yl)biphényl-4-yl)méthyl]-2-imidazolin-5-one de formule:

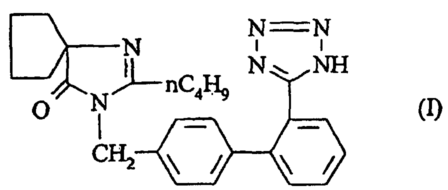

Ce composé et son mode de préparation ont été décrits pour la première fois dans le brevet européen EP 454 511. Le composé de formule (I) est un antagoniste de l'angiotensine II utile dans le traitement des maladies cardiovasculaires comme l'hypertension, l'insuffisance cardiaque l'arythmie cardiaque, dans le traitement des maladies du système nerveux central, dans le traitement du glaucome, de la rétinopathie diabétique et dans le traitement de l'insuffisance rénale et la néphropathie diabétique.

La dénomination commune du composé de formule (I) est irbesartan et le terme irbesartan est utilisé dans cette description et dans les revendications pour se référer au composés de formule (I).

La demande de brevet européenne EP 708 103 décrit l'existence de 2 formes cristallines pour l'irbesartan:
- l'une appelée forme A est celle obtenue par cristallisation dans un solvant contenant moins d'environ 10% en volume d'eau,
- l'autre appelée forme B est obtenue par cristallisation dans un solvant miscible à l'eau contenant plus d'environ 10 % d'eau.

Chacune de ces deux formes est caractérisée par un profil de diffraction des rayons X particulier.

On décrit dans la demande de brevet EP 708 103 que la forme B est une forme tautomérique ; de plus l'article de M. Bauer et al. dans J.Chem. Soc. Perkin Trans. 2, 1998, 475-481, étudie par RMN la desmotropie de l'ibersartan.

Il est indiqué dans la demande de brevet EP 708 103 que l'irbesartan dans la forme A se présente sous forme d'aiguilles stables, non hygroscopiques et présentant une électrostaticité élevée. Dans la suite de la présente description on désigne par le terme "habitus aiguille" cet habitus cristallin de l'irbesartan forme A.

On a également constaté que ces cristaux d'habitus aiguille sont difficiles à filtrer et à sécher et qu'ils présentent une mauvaise coulabilité.

On a maintenant trouvé un nouvel habitus cristallin de la forme A, caractérisé en ce que le rapport entre la longueur et la largeur des cristaux est compris entre 1:1 1 et 10:1, de préférence entre 1:1 et 5:1. Ce nouvel habitus cristallin de la forme A de l'irbesartan

| | |
|---|---|
| 4,61 | 15,90 |
| 4,49 | 14,73 |
| 4,36 | 9,86 |
| 4,17 | 62,84 |
| 4,07 | 15,39 |
| 3,97 | 30,34 |
| 3,88 | 14,32 |
| 3,83 | 13,56 |
| 3,75 | 37,28 |
| 3,53 | 26,48 |
| 3,46 | 12,42 |
| 3,40 | 27,88 |
| 3,27 | 11,03 |
| 3,18 | 10,42 |
| 3,15 | 7,28 |
| 3,12 | 6,11 |
| 3,05 | 15,50 |
| 3,01 | 9,49 |
| 2,81 | 7,11 |
| 2,78 | 9,40 |

Ce profil de diffraction est celui de la forme A de l'irbesartan décrit dans EP 708 103.

La chargeabilité de la poudre est mesurée par tribogénération : la poudre est soumise à une forte vibration pendant laquelle elle se charge sur elle-même, elle est ensuite versée dans une cage de Faraday reliée à un électromètre très sensible. La chargeabilité mesurée varie entre 0 et -10 nanocoulomb/g. A titre de comparaison, les cristaux d'irbesartan forme A en aiguilles présentent une chargeabilité, mesurée par le même procédé, comprise entre -30 et -40 nanocoulomb/g.

Ainsi la présente invention a pour objet un composé cristallin de formule : 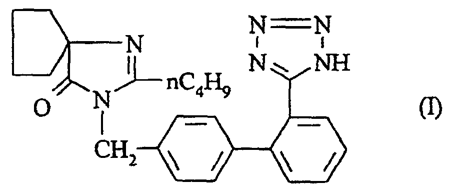 ayant un habitus cristallin tel que le rapport entre la longueur et la largeur des cristaux soit compris entre 1:1 et 10:1, préférentiellement entre 1:1 et 5:1 et dont la chargeabilité mesurée par tribogénération varie entre 0 et -10 nanocoulomb/g.

Plus particulièrement, l'invention a pour objet une nouvelle forme cristalline d'irbesartan forme A caractérisée en ce que le rapport entre la longueur et la largeur des cristaux est compris entre 1:1 et 5:1 et dont la chargeabilité mesurée par tribogénération varie entre 0 et -10 nanocoulomb/g.

La densité tassée des cristaux d'irbesartan présentant le nouvel habitus cristallin (180 coups) mesurée sur un appareil Hosokawa est d'environ 0,5 kg/m³ alors que celle des cristaux de forme A en aiguilles est d'environ 0,35 kg/m³.

L'indice de coulabilité est calculé d'après la méthode de Carr (R Carr : Chemical Engineering, January 18, 1965, page 163-168) et prend en compte les résultats de quatre valeurs expérimentales : compressibilité, angle de repos, angle de spatule et

On a maintenant trouvé un nouvel habitus cristallin de la forme A, caractérisé en ce que le rapport entre la longueur et la largeur des cristaux est compris entre 1:1 et 10:1, de préférence entre 1:1 1 et 5:1. Ce nouvel habitus cristallin de la forme A de l'irbesartan sera qualifié par le terme "habitus brique" de l'irbesartan dans la suite de la présente description.

La présente invention a également pour objet les procédés permettant d'obtenir les cristaux d'irbesartan de forme A présentant le nouvel habitus cristallin selon lequel le rapport entre la longueur et la largeur des cristaux est compris entre 1:1 1 et 10:1, de préférence entre 1:1 1 et 5:1.

Plus ce rapport est élevé, plus les aiguilles sont longues par rapport à leur largeur, ainsi une amélioration de ce rapport signifie une diminution dudit rapport. Il est préférable que ce rapport diminue de telle sorte qu'il soit compris entre 1:1 et 10:1, préférentiellement entre 1:1 et 5:1.

L'amélioration de ce rapport signifie que les cristaux ont moins tendance à se casser ou à s'agglomérer quand ils sont humides, qu'ils peuvent être filtrés et séchés plus vite, qu'ils sont plus facilement manipulables quand ils sont secs.

Les procédés selon l'invention n'ont pas d'effet sur le polymorphisme.

Les cristaux d'irbesartan dans l'habitus brique présentent les caractéristiques physico-chimiques décrites ci-après.

, Le profil de diffraction des rayons X de la poudre (angle de diffraction) a été établi avec un diffractomètre Siemens D 500 TT, les raies significatives sont consignées dans la Tableau I ci-après :

**Tableau I**

| d | I/I₀ |
|---|---|
| 11,22 | 100,00 |
| 7,90 | 12,02 |
| 7,52 | 13,79 |
| 7,23 | 18,60 |
| 6,27 | 20,14 |
| 6,09 | 6,47 |
| 5,86 | 7,42 |
| 5,60 | 98,76 |
| 5,41 | 19,45 |
| 5,05 | 24,67 |
| 4,97 | 20,36 |
| 4,91 | 12,92 |

de spatule et cohésion. Cet indice est d'environ 30 pour les cristaux d'habitus brique alors qu'il est d'environ 10 pour les cristaux d'habitus aiguille.

On constate que la résistivité, l'énergie minimum d'inflammation, la température minimum d'inflammation, les résultats du test de friction et la gravité de l'explosion, mesurés en sphère de 20 litres, sont proches pour les deux habitus cristallins de la forme A de l'irbesartan.

Le fait que les cristaux d'irbesartan d'habitus brique aient une chargeabilité diminuée, c'est à dire une tendance diminuée à stocker les charges électrostatiques, signifie que ces cristaux sont manipulables plus facilement et de façon plus sûre.

L'augmentation de 50 % de la densité tassée et de l'indice de coulabilité de l'habitus brique vis-à-vis de l'habitus aiguille représente une amélioration qui se traduit à la fois au niveau de la maniabilité chimique du produit et de leur utilisation pour la préparation de formes pharmaceutiques.

Selon la présente invention, on peut préparer l'irbesartan d'habitus brique en utilisant un procédé caractérisé en ce que l'on soumet une suspension cristalline d'irbesartan forme A d'habitus aiguille à au moins un épisode de sonication et au moins un épisode d'oscillation de température.

Ainsi l'épisode de sonication peut être soit suivi, soit précédé par l'épisode d'oscillation de température.

On peut aussi concevoir que l'épisode de sonication soit conduit simultanément à l'épisode d'oscillation de température. Selon l'invention un épisode de sonication peut également être conduit entre 2 phases d'oscillation de température.

De plus, les épisodes de sonication et/ou d'oscillation de température peuvent être répétés indépendamment les uns des autres.

Préférentiellement, un épisode de sonication est précédé par un épisode d'oscillation de température et plus particulièrement un épisode de sonication est conduit entre 2 épisodes d'oscillation de température.

Le terme "suspension cristalline" utilisé dans la présente description se réfère à une suspension d'irbesartan préparée selon des méthodes connues par l'homme du métier. Par exemple, la suspension cristalline peut être préparée en faisant croître des cristaux d'irbesartan dans un solvant organique par exemple un alcool tel que l'isopropanol, pour préparer une solution sursaturée d'irbesartan et en refroidissant à une température à laquelle la sursaturation est comprise entre 0 % et 50 %. La solution sursaturée est alors ensemencée avec 1 % à 10 % de semences cristallines d'irbesartan d'habitus brique, les semences cristallines provenant d'un lot précédent. Cependant les semences, peuvent également être engendrées en soumettant de façon répétée la suspension cristalline à des épisodes d'oscillation de température et de sonication jusqu'à ce que des cristaux d'habitus brique soient obtenus. La solution ensemencée est alors refroidie à température ambiante pour former la suspension cristalline. Ladite suspension cristalline est alors utilisée selon l'invention.

Selon la présente invention, un épisode de sonication consiste à soumettre la suspension cristalline à une énergie de sonication dont la fréquence est d'environ 16 kHz à 10 MHz. Il semble que l'épisode de sonication limite la croissance selon la longueur des aiguilles en les cassant et modifie le caractère des surfaces cristallines de telles sortes que les zones capables d'accumuler les charges électrostatiques soient diminuées. On peut utiliser des méthodes de sonication soit par lots, soit de façon semi-continue, soit de façon continue.

Pour la sonication par lots, on insère une sonde à ultrasons dans la suspension cristalline placée dans un cristalliseur.

L'épisode de sonication peut aussi être conduit en continu ou en semi-continu en pompant la suspension cristalline d'irbesartan à travers une cellule de sonication avec un débit d'environ 10 litres/min/KW à 20 litres/min/KW ; avec une pression d'environ 0 psig à 100 psig ; avec une énergie d'environ 10 000 joule/litre à 30 000 joule/litre et à une fréquence d'environ 16 kHz à 10 MHz. De préférence, le débit est compris entre 16 litre/min/KW et 18 litre/min/KW ; la pression est comprise entre 0 psig et 20 psig ; l'énergie est comprise entre 16 000 et 25 000 joule/litre et la fréquence est d'environ 20 kHz.

Les paramètres de sonication ci-dessus tels que le débit, la pression et la fréquence varient en fonction du résultat attendu en terme de rapport entre la longueur et la largeur des cristaux préparés.

L'épisode d'oscillation de température comprend une phase de chauffage et une phase de refroidissement. Selon l'invention il comprend au moins une phase de chauffage et au moins une phase de refroidissement dans n'importe quel ordre. Il est préférable qu'une phase de chauffage soit associée à une phase de refroidissement et même que ladite phase de chauffage précède ladite phase de refroidissement. Il est probable que l'oscillation de température contribue au contrôle de la bonne distribution de la taille des particules ; en particulier il tend à dissoudre les particules les plus fines, et à faire de la croissance sur les plus grosses particules.

L'oscillation de température est conduite en chauffant et en refroidissant une suspension cristalline à des températures préétablies. La phase de chauffage est menée en chauffant jusqu'à environ 20°C à 100°C. De façon préférentielle, la phase de chauffage est menée à une température telle qu'environ 15 % à 25 % soit dissous en 60 minutes, plus particulièrement environ 20 % des cristaux dissous en 60 minutes. La phase de refroidissement de l'épisode d'oscillation de température est généralement conduite entre 100°C et -20°C. Préférentiellement, la phase de refroidissement est conduite à une température comprise entre -5°C et 20°C pendant environ 0 à 60 minutes ; plus particulièrement entre 0 et 5°C pendant environ 0 à 60 minutes.

Il faut noter que la température choisie pour la phase de refroidissement de l'épisode d'oscillation de température est inférieure à la température choisie pour la phase de chauffage correspondante. Les phases de chauffage et de refroidissement peuvent être répétées indépendamment, autant de fois que nécessaire et les paramètres spécifiques peuvent être modifiés pour obtenir le produit souhaité.

Par exemple, on peut allonger la phase de chauffage et raccourcir la phase de refroidissement pour générer des cristaux plus petits ou bien on peut raccourcir la phase de chauffage et allonger la phase de refroidissement pour générer des cristaux plus larges. Le nombre de phases de chauffage et de refroidissement dépend également du résultat souhaité. D'une façon générale, si le nombre de phases de chauffage et de refroidissement augmente, l'aspect des cristaux s'améliore et le rapport entre la longueur et la largeur tend vers 1:1.

Le contrôle des paramètres de sonication et d'oscillation de température permet de contrôler la distribution de la taille des particules et le rapport entre la longueur et la largeur des cristaux finaux.

Le procédé décrit ci-dessus pour modifier l'habitus cristallin de l'irbesartan utilisant la sonication présente des difficultés de mise en oeuvre sur le plan industriel. En effet, l'émetteur d'ultrasons a une efficacité qui diminue au delà de quelques centimètres dudit émetteur ; de plus, lorsque l'on travaille en continu, cette efficacité diminue si l'on augmente la vitesse de passage de la suspension cristalline traitée.

Aussi, pour traiter de grands volumes, le temps d'application est très long. Par ailleurs, les ultrasons à forte puissance provoquent une usure prématurée des métaux et des soudures des appareils utilisés.

Un autre procédé pour modifier l'habitus cristallin de l'irbesartan forme A utilise le broyage en voie humide, c'est à dire le cisaillement mécanique des cristaux d'habitus aiguille pour les transformer en cristaux d'habitus brique. Ce procédé présente l'avantage d'être facilement applicable au traitement de quantités industrielles de produit.

Ainsi, selon un autre de ses aspects, la présente invention est relative à un procédé de préparation de l'irbesartan d'habitus brique caractérisé en ce qu'il contient les étapes consistant à :
a) préparer une solution d'irbesartan forme A dans un alcool, dans des conditions de concentration et de température permettant la solubilité totale de l'irbesartan ;
b) refroidir ladite solution à une température choisie en fonction de la concentration de la solution de telle sorte que la solution soit dans la zone métastable ;
c) ensemencer par des cristaux d'irbesartan d'habitus brique;
d) refroidir la solution d'irbesartan à une température comprise environ entre 20°C et 5°C ;
e) soumettre la suspension cristalline ainsi formée à un cisaillement mécanique par une machine à cisailler ;
f) réchauffer la suspension cristalline à une température comprise environ entre 40°C et 60°C pour dissoudre les fines particules ;
g) refroidir la suspension cristalline à une température comprise environ entre 20°C et 5°C;
h) filtrer les cristaux d'habitus brique ainsi formés.

Selon la présente invention, on utilise une solution d'irbesartan dans l'alcool, par exemple l'éthanol ou, de préférence, l'isopropanol.

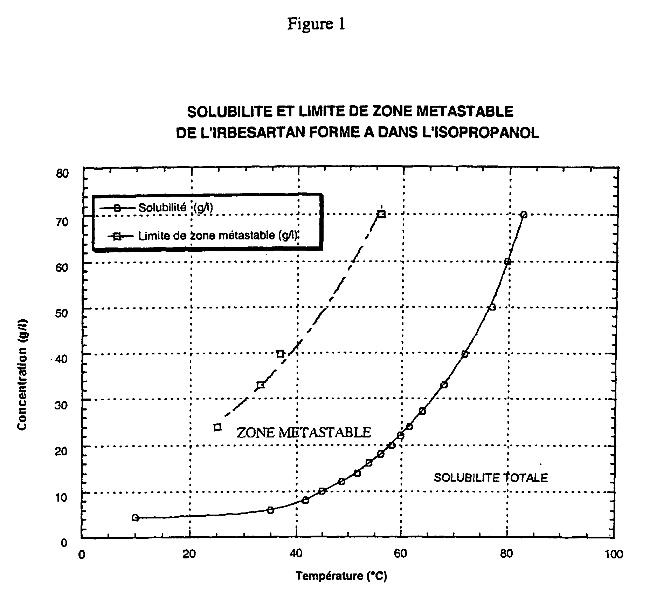

La figure 1 indique, pour une solution d'irbesartan forme A dans l'isopropanol, les conditions de solubilité totale, en fonction de la concentration en g/litre et de la température en °C. Elle indique également les limites de la zone métastable pour une solution contenant 25 g/litre à 70 g/litre d'irbesartan.

Ainsi pour une solution d'irbesartan dans l'isopropanol contenant environ 50 g/litre à 70 g/litre la température d'ensemencement varie de 45°C à 80°C pour que la solution reste dans la zone métastable.

On peut ensemencer la solution d'irbesartan par des cristaux d'irbesartan d'habitus brique à un moment quelconque du refroidissement de la solution, lorsque celle-ci est dans la zone métastable. La température d'ensemencement est comprise entre 25°C et 80°C, selon la concentration de la solution. La proportion de semence cristalline incorporée peut être comprise entre 1 % et 25 %, préférentiellement entre 10 % et 20 %. Après l'ensemencement, on peut maintenir la température constante pendant une durée comprise entre quelques minutes et 2 heures, préférentiellement pendant une demie-heure à une heure.

Aux étapes b) et d) on procède avantageusement au refroidissement selon une vitesse de refroidissement régulière d'environ 5°C à 20°C par heure, préférentiellement voisine de 10°C par heure.

A l'étape e) le cisaillement mécanique est effectué préférentiellement avec une machine ayant une vitesse de rotation d'environ 10 000 à 15 000 tours/minute.

Des machines possédant de telles caractéristiques sont par exemple du type Turrax® , vendues par IKA-Werke (Allemagne). Certaines de ces machines sont adaptées au traitement de quantités industrielles allant jusqu'à permettre un débit de 100 m³/heure. Pour le procédé selon l'invention et à un stade industriel, un débit compris environ entre 500 litres/heure et 4 m³/heure est préféré, dans un réacteur de 2m³.

Le cisaillement mécanique de l'étape e) peut être conduit soit en plaçant la machine à cisailler dans le réacteur contenant la suspension cristalline, soit en faisant passer la suspension cristalline en continu dans la machine à cisailler. Dans ce cas, le débit de la machine est ajusté en fonction du rapport entre la longueur et la largeur désiré pour les cristaux d'habitus brique formés.

Eventuellement, afin d'améliorer le rendement en cristaux d'habitus brique, on peut répéter les étapes e), f) et g) avant de filtrer les cristaux d'habitus brique formés et de les sécher.

La présente invention a également pour objet les compositions pharmaceutiques contenant en tant que principe actif l'irbesartan d'habitus brique, c'est à dire l'irbesartan forme A, présentant un nouvel habitus cristallin. Ces compositions pharmaceutiques peuvent être préparées selon la description de la demande de brevet EP 747 050.

Les formulations préparées avec l'habitus brique peuvent contenir jusqu'à environ 80 % en poids d'irbesartan ou environ 85 % en poids d'irbesartan associé à un diurétique, par exemple l'hydrochlorothiazide. Ces formulations peuvent être préparées industriellement, par exemple sous forme de comprimés ou gélules, selon des procédés connus, par exemple par granulation voie humide, par granulation voie sèche, par compression directe.

Par compression, on obtient des comprimés uniformes en poids et en contenu qui possèdent des propriétés physiques appropriées à un développement industriel.

### EXEMPLE 1

### A Préparation de l'irbesartan forme A.

L'irbesartan est préparé selon le mode opératoire décrit dans le brevet européen EP 454 511.

### 1) 2-n-butyl-4-spirocyclopentane-2-imidazoline-5-one.

L'ester éthylique de l'acide amino-1-cyclopentane carboxylique est préparé selon Adkins et Billica (J. Amer. Chem. Soc., 1948, 70, 3121).

Le valérimidate d'éthyle est préparé selon Mac Elvain (J. Amer. Chem. Soc., 1942, 64, 1825-1827) puis libéré de son chlorhydrate par action du carbonate de potassium et extraction par le chlorure de méthylène.

L'ester éthylique de l'acide amino-1-cyclopentane carboxylique (1,57 g) et le valérimidate d'éthyle (1,56 g) sont dissous dans 12 ml de xylène contenant 6 gouttes d'acide acétique. Après 6 heures et demi de chauffage à reflux, le milieu réactionnel est concentré sous vide puis le résidu est chromatographié sur gel de silice en éluant par un mélange chloroforme/méthanol/acide acétique (94/4/2 ; v/v/v). La fraction contenant le produit attendu est évaporée plusieurs fois en présence de xylène puis de benzène pour éliminer l'acide acétique. On obtient 1,91 g de produit sous forme d'une huile épaisse.
IR (CHCl₃) : 1720 cm⁻¹ : C = O ; 1635 cm⁻¹ : C = N.

Remarque : le fait de ne pas voir de bande entre 1500 et 1600 cm⁻¹ indique que, dans la solution de chloroforme, le produit est une imidazoline-5-one.
Spectre RMN : 0,92 ppm : t : 3H : CH₃ (nBu) ; 1,35 ppm : sext : 2H : CH₃CH₂-.

1,50-1,93 ppm : m : 10H : CH₃-CH₂-CH₂, et cyclopentane ;
2,33 ppm : t : 2H : CH₃-CH₂-CH₂-CH₂- ; 10,7 ppm : m : NH.
Spectre de masse : MH⁺ : 195.

La 2-n-butyl-4-spirocyclopentane-2-imidazoline-5-one préparé à l'étape A, peut également être obtenue selon un autre mode opératoire décrit ci-dessous, en utilisant comme produit de départ la cyclopentanone.

### i) 1-aminocyclopentanenitrile.

Cette étape est réalisée selon A. Strecker (Org. Synth., 1955, 3).

Dans un ballon on dissout 1,97 g de cyanure de sodium dans 3,9 ml d'eau et on ajoute une solution contenant 2,33 g de chlorure d'ammonium dans 5,9 ml d'eau et 3,5 ml d'ammoniaque à 20 %, enfin on ajoute dans le ballon 3 g de cyclopentanone dans 3,8 ml de méthanol. Après 1 heure et demie sous agitation, on porte à 60°C pendant 45 minutes puis on arrête le chauffage, maintient l'agitation pendant 45 minutes puis refroidit à 25°C. On extrait plusieurs fois par du chlorure de méthylène. On sèche sur sulfate de sodium, filtre et concentre sous vide. On obtient 4 g du produit attendu sous forme huileuse.

Le 1-aminocyclopentanenitrile obtenu est mis en solution dans 300 ml d'acétone et l'on ajoute, sous agitation, une solution de 2,25 g d'acide oxalique dihydraté dans 200 ml d'acétone. Le précipité formé est essoré, lavé avec de l'acétone puis séché.
m=4,71 g.
Fc = 220°C.

Ce composé est l'hemioxalate de 1-aminocyclopentanenitrile.

### ii) 1-aminocyclopentaneacétamide.

Cette étape est réalisée selon J. Zabicky, (The Chemistry of Amides, Intersciences, New York, 1970, 119).

5,1 g de l'oxalate obtenu à l'étape précédente sont traités en 45 minutes et sous agitation par 7,65 ml d'acide sulfurique concentré (d = 1,84). On observe un dégagement gazeux, la température augmente jusqu'à 100°C. On refroidit vers 35°C et verse sur un mélange glace-ammoniaque concentrée, (10 g/2,8 ml). La suspension formée est extraite 6 fois de suite avec du chloroforme contenant 5 % de méthanol. On ajoute 3 ml d'ammoniaque (d = 0,92) à la phase aqueuse et extrait à nouveau par du chloroforme contenant du méthanol (1/0,5 ; v/v). Les phases organiques réunies sont séchées sur sulfate de sodium, on filtre et concentre. Le produit attendu est obtenu sous forme d'un solide blanc.
m = 3,79 g.
Fc = 95°C.

Les résultats de l'analyse et le spectre IR permettent de confirmer la structure.

### iii) 2-n-butyl-4-spirocyclopentane-2-imidazoline-5-one.

Cette étape est réalisée selon H. Takenaka et al., Heterocycles, 1989, 29 (6), 1185-89.

3 g du composé préparé à l'étape précédente sont placés dans 70 ml de THF anhydre et 3,3 ml de triéthylamine et l'on ajoute, sous agitation, 3 ml de chlorure de valéryle dans 10 ml de THF anhydre. Il se forme une suspension blanche. Le composé intermédiaire formé, mais non isolé, est le (N-valéryl)-1-aminocyclopentane carboxamide. On ajoute 6 g de potasse en pastille, 7 ml d'eau et 16 ml de méthanol. On chauffe à reflux pendant 2 heures et demie puis l'on ajoute 9 g de chlorure d'ammonium. Après 15 minutes d'agitation, on concentre sous vide. Le résidu obtenu est repris par 40 ml d'eau et extrait par 10 ml d'acétate d'éthyle puis 2 fois par 5 ml d'acétate d'éthyle. Les phases organiques réunies sont séchées sur sulfate de sodium et filtrées. Le filtrat est concentré à sec. On obtient 4,85 g du produit attendu. Le spectre de RMN est semblable à celui décrit précédemment. On peut préparer le chlorhydrate de ce composé par addition d'acide chlorhydrique concentré. Le chlorhydrate fond à 240°C en se sublimant.

### 2) 1-[(2'-Cyanobiphényl-4-yl)méthyl]-2-n-butyl-4-spirocyclopentane-2-imidazoline-5-one.

On prépare sous atmosphère d'azote un mélange contenant 250 mg d'hydrure de sodium (dispersé à 80 % dans l'huile minérale) et 5 ml de DMF et on ajoute goutte à goutte une solution contenant 0,97 g de 2-n-butyl-4-spirocyclopentane-2-imidazoline-5-one dans 10 ml de DMF. On agite 30 minutes à TA puis on ajoute une solution de 1,5 g de 4-bromométhyl-2-cyanobiphényle dans 10 ml de DMF. Après 1 heure d'agitation à TA, on évapore le DMF sous pression réduite puis on reprend le résidu par de l'acétate d'éthyle, lave la phase organique avec de l'eau puis sèche sur sulfate de sodium, filtre et évapore, le résidu est chromatographié sur gel de silice en éluant par un mélange DCM/acétate d'éthyle (9/1 ; v/v). On récupère 1,68 g du produit attendu. Fc = 92-93°C.

### 3) 2-n-Butyl-4-spirocyclopentane-1-[2'-(triphénylméthyltétrazol-5-yl)biphényl-4-ylméthyl]-2-imidazoline-5-one.

1,56 g du produit précédent, 2,6 g d'azide de tributylétain et 30 ml de xylène sont chauffés à reflux pendant 66 heures. Le xylène est ensuite évaporé et le résidu est dissous dans 20 ml de DCM et 5 ml de THF, en ajoutant 0,8 ml de soude 10N et, après 30 minutes d'agitation, 2,5 g de chlorure de trityle et on laisse sous agitation pendant 26 heures. Après évaporation des solvants, le résidu est repris par de l'acétate d'éthyle et lavé à l'eau par une solution de sulfate acide de potassium à 3 % et de d'eau. On sèche et évapore. Le résidu est chromatographié sur alumine en éluant sur le mélange hexane/acétate d'éthyle (9/1 : v/v). On obtient 1,97 g du produit attendu. Fc = 150-152°C.

### 4) 2-n-Butyl-4-spirocyclopentane-1-[(2'-(tétrazol-5-yl)biphényl-4-yl)méthyl]-2-imidazoline-5-one.

1,96 g du produit préparé à l'étape précédente sont dissous dans 10 ml de méthanol et 10 ml de THF. Après refroidissement du milieu réactionnel à 5°C, on ajoute 1,5 ml d'acide chlorhydrique 4N et on agite pendant 3 heures à TA et 1 heure à 30°C. Après évaporation des solvants, le résidu est repris par de l'eau et l'on porte à pH 12 par addition de soude 10N. La phase aqueuse est extraite par de l'éther, du toluène et à nouveau de l'éther. On acidifie la phase aqueuse jusqu'à pH 2 par addition d'acide chlorhydrique 1N, puis on extrait à l'acétate d'éthyle, sèche sur Na₂SO₄ et évapore. Le solide blanc obtenu est séché à 50°C sous 0,05 mm de mercure. On obtient 840 mg du produit attendu. Fc = 180-181°C.
Spectre de RMN : 0,75 ppm : t : 3H : CH₃ (nBu) ;
1,10 ppm : sext : 2H : CH₃-CH₂- ; 1,20 ppm : quint : 2H : CH₃-CH₂-CH₂-;
1,5-2 ppm : m : 8H : -C₅H₈ ; 2,2 ppm : t : 2H : CH₃-CH₂-CH₂-CH₂;
4,6 ppm : s : 2H : CH₂-C₆H₄- ; 7 ppm : s : 4H : CH₂-C₆H₄- ;
7,35-7,7 ppm : m : 4H : H_{3', 4', 5', 6'} aromatiques.

L'étude N.O.E. confirme la position de la substitution 5-one sur l'imidazole.

Les cristaux formés peuvent être caractérisés par leur spectre de diffraction des rayons X (tableau 1) et correspondent à l'irbesartan forme A.

| d | I/I₀ |
|---|---|
| 11,22 | 100,00 |
| 7,90 | 12,02 |
| 7,52 | 13,79 |
| 7,23 | 18,60 |
| 6,27 | 20,14 |
| 6,09 | 6,47 |
| 5,86 | 7,42 |
| 5,60 | 98,76 |
| 5,41 | 19,45 |
| 5,05 | 24,67 |
| 4,97 | 20,36 |
| 4,91 | 12,92 |
| 4,80 | 27,33 |
| 4,61 | 15,90 |
| 4,49 | 14,73 |
| 4,36 | 9,86 |
| 4,17 | 62,84 |
| 4,07 | 15,39 |
| 3,97 | 30,34 |
| 3,88 | 14,32 |
| 3,83 | 13,56 |
| 3,75 | 37,28 |
| 3,53 | 26,48 |
| 3,46 | 12,42 |
| 3,40 | 27,88 |
| 3,27 | 11,03 |
| 3,18 | 10,42 |
| 3,15 | 7,28 |
| 3,12 | 6,11 |
| 3,05 | 15,50 |
| 3,01 | 9,49 |
| 2,81 | 7,11 |
| 2,78 | 9,40 |

Les cristaux ainsi obtenus peuvent être recristallisés de la façon suivante.

A 840 mg de produit obtenu, on ajoute 15 ml d'isopropanol et on chauffe le mélange jusqu'à dissolution complète. On laisse revenir à température ambiante, puis on filtre les cristaux formés, on les lave à l'eau et on les sèche. On obtient 805 mg.d'irbesartan forme A.

### B Préparation des semences cristallines.

Les cristaux utilisés ensuite comme semences, sont préparés selon la procédure suivante.

### Cycle I.

Un ballon tricol équipé d'un agitateur mécanique est chargé avec 200 ml d'alcool isopropylique et 9,40 g du composé obtenu à l'étape A. La suspension cristalline est chauffée à 77,0°C, sous agitation (environ 100 tours/minutes) pour une dissolution complète. La solution est refroidie à 73,0°C et on ajoute 0,09 mg supplémentaire du composé de l'étape A pour initier la cristallisation. La suspension cristalline est refroidie à 20,0°C pendant 20 minutes. La suspension est soumise à une sonication pendant 600 secondes avec une puissance de 10-15 watts en utilisant une sonde de sonication de 0,63 cm O.D.

### Cycle II.

La suspension cristalline est chauffée à 74,0°C, ce qui dissout environ 93 % des cristaux, laissant seulement les cristaux les plus grands pour la prochaine cristallisation.

Le mélange est refroidi à 20,0°C pendant 180 minutes suivant la diminution cubique de température décrite ci-après :

| Temps, minutes | Température, °C |
|---|---|
| 0 | 74,0 |
| 30 | 73,8 |
| 60 | 72,0 |
| 90 | 67,3 |
| 120 | 58,0 |
| 150 | 42,8 |
| 180 | 20,0 |

Quand la température de 20,0°C est atteinte, le milieu réactionnel est soumis à sonication pendant 600 secondes avec une puissance de 10-15 watts.

### Cycle III.

La suspension cristalline est chauffée à 74,0°C. Comme dans le cycle II, elle est refroidie à 20,0°C pendant 180 minutes en suivant la diminution cubique de température décrite ci-dessus. Quand la température de 20,0°C est atteinte, la suspension cristalline est soumise à sonication pendant 600 secondes avec une puissance de 10 à 15 watts.

### Cycle IV.

La suspension cristalline est chauffée à 74,0°C. Comme dans le cycle II, elle est refroidie à 20,0°C pendant 180 minutes en suivant la diminution cubique de température décrite ci-dessus. Quand la température de 20,0°C est atteinte, la suspension cristalline est soumise à sonication pendant 600 secondes avec une puissance de 10 à 15 watts.

### Cycle V.

La suspension cristalline est chauffée à 74,0°C. Comme dans le cycle II, elle est refroidie à 20,0°C pendant 180 minutes en suivant la diminution cubique de température décrite ci-dessus. Quand la température de 20,0°C est atteinte, la suspension cristalline est soumise à sonication pendant 600 secondes avec une puissance de 10 à 15 watts.

### Cycle VI.

La suspension cristalline est chauffée à 74,0°C. Comme dans le cycle II, elle est refroidie à 20,0°C pendant 180 minutes en suivant la diminution cubique de température décrite ci-dessus. La suspension cristalline est refroidie à 5,0°C et le produit est filtré dans un Buchner et séché sous vide à 70°C pendant une nuit pour donner les semences cristallines.

### C Procédure de cristallisation.

515 g du composé de l'étape A sont mélangés à 10,95 litres d'isopropanol pour former la suspension cristalline. Celle-ci est chauffée à 80°C pour dissoudre tout le solide. La suspension cristalline est alors refroidie jusqu'à 20°C en suivant la diminution cubique de température décrite ci-dessus pendant 4 heures et en ajoutant à 73°C, 5,13 g de semences cristallines, obtenues à l'étape B. On introduit une sonde de sonication de 1,27 cm O.D. pendant 10 minutes avec une puissance de 125 W. La solution est chauffée à nouveau à 73°C pour dissoudre les petits cristaux, puis refroidie à 20°C pendant 4 heures selon la diminution cubique de température décrite ci-dessus.

La solution est alors soumise à sonication pendant 10 minutes avec une puissance de 125 W. La solution est à nouveau chauffée à 73°C pour dissoudre les petits cristaux. La solution est refroidie à 2°C, en utilisant la diminution cubique de température décrite ci-dessus pendant 6 heures puis la solution est maintenue à 2°C pendant 1heure. Le milieu réactionnel est filtré pour former un filtrat humide. Celui-ci est séché à 50°C sous vide pendant une nuit. 513,4g du produit sec sont obtenus présentant une relation largeur:longueur de 1:2 à 1:5.

### EXEMPLE 2

### A) Préparation de la solution d'irbesartan forme A.

On procède selon le mode opératoire décrit à l'exemple 1, étape A. Dans un réacteur de 2000 l, on charge 116 kg d'irbesartan et 1585 l d'isopropanol puis on chauffe à reflux pendant 30 minutes pour obtenir une dissolution totale. On filtre à chaud pour éliminer les particules insolubles vers un autre réacteur, en passant sur une cartouche de 0,6 µm de seuil de coupure. La solution filtrée est à nouveau chauffée à reflux pour dissoudre les germes de cristaux éventuellement présents puis elle est refroidie à 80°C sous agitation d'environ 50 tours par minute.

### B) Préparation des semences cristallines.

Les semences cristallines sont obtenues en laboratoire par des étapes successives de chauffage et de refroidissement d'une solution d'irbesartan forme A dans l'isopropanol, la solution subissant un passage dans la machine à cisaillement (Turrax® ) après chaque refroidissement.

### C) Procédure de cristallisation

a) On prépare une suspension de semences cristallines contenant 17,4 kg dans 33 1 d'isopropanol et on l'introduit en une fois dans la solution préparée à l'étape A et maintenue à 80°C pendant 1 heure. On refroidit la température du réacteur jusqu'à 20°C selon une vitesse de refroidissement régulière de 10°C par heure. On obtient une population de cristaux dont la longueur est 300 µm à 500 µm et la largeur 20 µm à 50 µm en fin de cristallisation soit un rapport de 25: 1 à 6:1.
b) La suspension cristalline est passée en 35 minutes (débit 4 m³/heure) dans une machine à cisailler Turrax® , référence IKA/DISPAX Reactor DRS 2/10, ayant une vitesse de rotation de 12 000 tours/minutes. On obtient des cristaux dont la longueur est 40 µm à 110 µm et la largeur 5 µm à 40 µm soit un rapport de 8:1 à 1:1. De nombreuses fines particules apparaissent également.
c) On réchauffe la température du réacteur à 50°C et on maintient à cette température 1 heure pour dissoudre les fines particules.
d) On refroidit la température du réacteur à 5°C selon une vitesse de refroidissement régulière de 10°C par heure puis on maintient une heure à cette température.
e). Par filtration, on obtient une population de cristaux d'habitus brique (longueur moyenne 30 µm, largeur moyenne 5 µm, rapport 6:1). Après séchage, on obtient 121 kg de cristaux d'habitus brique dont la teneur en isopropanol est inférieure à 1000 ppm.

### EXEMPLE 3

| Comprimé : formule centésimale | |
|---|---|
| Irbesartan d'habitus brique | 70 |
| cellulose microcristalline | 24,75 |
| croscarmellose de sodium | 3,75 |
| silice colloïdale hydratée | 0,75 |
| stéarate de magnésium | 0,75 |

### EXEMPLE 4

| Comprimé : formule centésimale | |
|---|---|
| Irbesartan d'habitus brique | 70 |
| cellulose microcristalline | 12,375 |
| croscarmellose de sodium | 3,75 |
| polyéthylène glycol | 12,375 |
| silice colloïdale hydratée | 0,75 |
| stéarate de magnésium | 0,75 |

### EXEMPLE 5

| Comprimé | |
|---|---|
| Irbesartan d'habitus brique | 75 mg |
| hydrochlorothiazide | 12,50 mg |
| cellulose microcristalline | 7,75 mg |
| croscarmellose de sodium | 3,25 mg |
| silice colloïdale hydratée | 0,75 mg |
| stéarate de magnésium pour un comprimé. | 0,75 mg |

### EXEMPLE 6

| Comprimé | |
|---|---|
| Irbesartan d'habitus brique | 150 mg |
| hydrochlorothiazide | 12,50 mg |
| cellulose microcristalline | 15,50 mg |
| croscarmellose de sodium | 6,50 mg |
| silice colloïdale hydratée | 1,50 mg |
| stéarate de magnésium | 1,50 mg |
| pour un comprimé. | |

## Revendications

1. Un composé cristallin de formule: 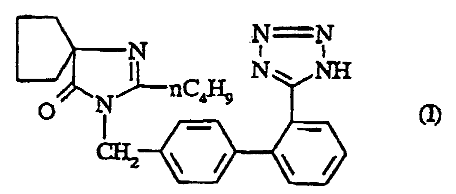 ayant un habitus cristallin tel que le rapport entre la longueur et la largeur des cristaux soit compris entre 1:1 et 10:1 et dont la chargeabilité mesurée par tribogénération varie entre 0 et -10 nanocoulomb/g.

2. Un composé cristallin selon la revendication 1 dans lequel le rapport entre la longueur et la largeur des cristaux est compris entre 1:1 et 5:1.

3. Une nouvelle forme cristalline d'irbesartan forme A **caractérisé en ce que** le rapport entre la longueur et la largeur des cristaux est compris entre 1 : 1 et 5 :1 et dont la chargeabilité mesurée par tribogénération varie entre 0 et -10 nanocoulomb/g.

4. Un procédé pour la préparation d'un composé selon l'une quelconque des revendications 1 à 3 **caractérisé en ce que** l'on soumet une suspension cristalline d'un composé de formule (I) à au moins un épisode de sonication et au moins un épisode d'oscillation de température.

5. Un procédé pour la préparation d'un composé selon l'une quelconque des revendications 1 à 3 **caractérisé en ce que** l'on soumet une suspension cristalline d'irbesartan forme A d'habitus aiguille à au moins un épisode de sonication et au moins un épisode d'oscillation de température.

6. Un procédé selon l'une quelconque des revendications 4 ou 5 dans lequel l'épisode d'oscillation de température comprend une phase de chauffage et une phase de refroidissement correspondante.

7. Un procédé selon la revendication 6 dans lequel la phase de chauffage précède la phase de refroidissement.

8. Un procédé selon la revendication 7 dans lequel l'épisode de sonication est suivi par un épisode d'oscillation de température.

9. Un procédé selon l'une quelconque des revendications 4 ou 5 dans lequel l'épisode de sonication est précédé par un épisode d'oscillation de température.

10. Un procédé selon l'une quelconque des revendications 4 ou 5 dans lequel l'épisode de sonication est conduit simultanément à l'épisode d'oscillation de température.

11. Un procédé selon l'une quelconque des revendications 4 ou 5 dans lequel un épisode de sonication est conduit entre 2 épisodes d'oscillation de température.

12. Un procédé selon l'une quelconque des revendications 4 ou 5 dans lequel les épisodes de sonication et/ou d'oscillation de température sont répétés indépendamment.

13. Un procédé selon l'une quelconque des revendications 4 ou 5 dans lequel la sonication est effectuée soit par lots, soit de façon semi-continue, soit de façon continue.

14. Un procédé selon la revendication 7 dans lequel la phase de chauffage de l'épisode d'oscillation de température est conduit à une température environ comprise entre 20°C et 100°C.

15. Un procédé selon la revendication 7 dans lequel la phase de chauffage de l'épisode d'oscillation de température est conduite à une température telle qu'environ 15 % à 25 % des cristaux soient dissous en environ 60 minutes.

16. Un procédé selon la revendication 7 dans lequel la phase de refroidissement de l'épisode d'oscillation de température est conduit à une température environ comprise entre 100°C et -20°C.

17. Un procédé selon la revendication 7 dans lequel la phase de refroidissement de l'épisode d'oscillation de température est conduit à une température environ comprise entre -5°C et 20°C.

18. Un procédé selon la revendication 7 dans lequel la température choisie pour la phase de refroidissement de l'épisode d'oscillation de température est inférieure à la température choisie pour la phase de chauffage correspondante de l'épisode d'oscillation de température.

19. Un procédé selon la revendication 7 dans lequel la suspension critalline est ensemencée par des cristaux d'irbesartan dont le rapport entre la longueur et la largeur est compris entre 1:1 et 10:1.

20. Un procédé pour la préparation d'un composé selon l'une quelconque des revendications 1 à 3 **caractérisé en ce qu'**il contient les étapes consistant à :
a) préparer une solution d'irbesartan forme A d'habitus aiguille dans un alcool, dans des conditions de concentration et de température permettant la solubilité totale de l'irbesartan;
b) refroidir ladite solution à une température choisie en fonction de la concentration de la solution de telle sorte que la solution soit dans la zone métastable ;
c) ensemencer par des cristaux d'irbesartan d'habitus brique ;
d) refroidir la solution d'irbesartan à une température comprise environ entre 20°C et 5°C ;
e) soumettre la suspension cristalline ainsi formée à un cisaillement mécanique par une machine à cisailler ;
f) réchauffer la suspension cristalline à une température comprise environ entre 40°C et 60°C pour dissoudre les fines particules ;
g) refroidir la suspension cristalline à une température comprise environ entre 20°C et 5°C ;
h) filtrer les cristaux d'habitus brique ainsi formés.

21. , Un procédé selon la revendication 20 dans lequel, à l'étape a), l'irbesartan est en solution dans l'isopropanol.

22. Un procédé selon la revendication 20 dans lequel, à l'étape b), une solution contenant 50 g/litre à 70 g/litre d'irbesartan dans l'isopropanol est refroidie à une température variant entre 60°C et 80°C.

23. Un procède selon la revendication 20 dans lequel, à l'étape c), la solution est ensemencée par des cristaux d'irbesartan dont la rapport entre la longueur et la largeur est compris entre 1:1 et 10:1.

24. Un procédé selon la revendication 23 dans lequel la solution ensemencée est maintenue à une température comprise entre 80°C et 22°C pendant quelques minutes à environ 2 heures avant d'être refroidie.

25. Un procédé selon la revendication 21 dans lequel, aux étape b) et d), la vitesse de refroidissement est d'environ 5°C à 20°C par heure.

26. Un procédé selon la revendication 20 dans lequel, à l'étape e), le cisaillement mécanique s'effectue par une machine ayant une vitesse de rotation d'environ 10 000 tours/minutes à 15 000 tours/minutes.

27. Procédé selon la revendication 26 dans lequel le cisaillement mécanique de l'étape e) s'effectue soit en plaçant la machine à cisailler directement dans le réacteur, sait en faisant passer la suspension cristalline dans la machine à cisailler.

28. Composition pharmaceutique contenant un composé selon l'une quelconque des revendications 1 à 3 et des excipients pharmaceutiques convenables.

29. Composition pharmaceutique selon la revendication 28 contenant un diurétique associé à un composé selon l'une quelconque des revendications 1 à 3.

30. Composition pharmaceutique selon la revendication 29 dans laquelle le diurétique est l'hydrochlorothiazide.

## Patentansprüche

1. Kristalline Verbindung der Formel: 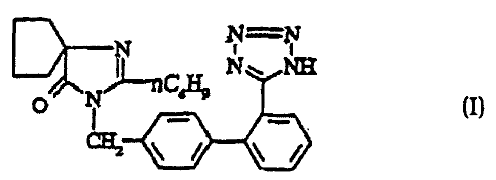 mit einem Kristallhabitus, bei dem das Verhältnis zwischen der Länge und der Breite der Kristalle entweder zwischen 1:1 und 10:1 liegt und deren durch Reibungsaufladung gemessene Ladefähigkeit zwischen 0 und -10 Nanocoulomb/g beträgt.

2. Kristalline Verbindung nach Anspruch 1, worin das Verhältnis von Länge zu Breite der Kristalle zwischen 1:1 1 und 5:1 liegt.

3. Neue Kristallform von Irbesartan der Form A, **dadurch gekennzeichnet, daß** das Verhältnis zwischen der Länge und der Breite der Kristalle zwischen 1:1 und 5:1 liegt und deren durch Reibungsladung gemessene Ladefähigkeit zwischen 0 und -10 Nanocoulomb/g liegt.

4. Verfahren zur Herstellung einer Verbindung nach einem der Ansprüche 1 bis 3, **dadurch gekennzeichnet, daß** man eine Kristallsuspension einer Verbindung der Formel (I) mindestens einer Schallbehandlung und mindestens einer Temperaturoszillationsbehandlung unterwirft.

5. Verfahren zur Herstellung einer Verbindung nach einem der Ansprüche 1 bis 3, **dadurch gekennzeichnet, daß** man eine Kristallsuspension von Irbesartan der Form A mit Nadelhabitus mindestens einer Schallbehandlung und mindestens einer Temperaturoszillationsbehandlung unterwirft.

6. Verfahren nach einem der Ansprüche 4 oder 5, worin die Temperaturoszillationsbehandlung eine Aufheizphase und eine entsprechende Abkühlphase umfaßt.

7. Verfahren nach Anspruch 6, worin die Aufheizphase der Abkühlphase vorausgeht.

8. Verfahren nach Anspruch 7, worin die Schallbehandlung von einer Temperaturoszillationsbehandlung gefolgt wird.

9. Verfahren nach einem der Ansprüche 4 oder 5, worin der Schallbehandlung eine Temperaturoszillationsbehandlung vorausgeht.

10. Verfahren nach einem der Ansprüche 4 oder 5, worin die Schallbehandlung gleichzeitig mit der Temperaturoszillationsbehandlung durchgeführt wird.

11. Verfahren nach einem der Ansprüche 4 oder 5, worin die Schallbehandlung zwischen zwei Temperaturoszillationsbehandlungen durchgeführt wird.

12. Verfahren nach einem der Ansprüche 4 oder 5, worin die Schallbehandlungen und/oder Temperaturoszillationsbehandlungen unabhängig voneinander wiederholt werden.

13. Verfahren nach einem der Ansprüche 4 oder 5, worin die Schallbehandlung entweder chargenweise oder halbkontinuierlich oder kontinuierlich erfolgt.

14. Verfahren nach Anspruch 7, worin die Aufheizphase der Temperaturoszillationsbehandlung bei einer Temperatur zwischen etwa 20°C und 100°C durchgeführt wird.

15. Verfahren nach Anspruch 7, worin die Aufheizphase der Temperaturoszillationsbehandlung bei einer solchen Temperatur durchgeführt wird, daß etwa 15 % bis 25 % der Kristalle in etwa 60 Minuten gelöst sind.

16. Verfahren nach Anspruch 7, worin die Abkühlphase der Temperaturoszillationsbehandlung bei einer Temperatur zwischen etwa 100°C und -20°C durchgeführt wird.

17. Verfahren nach Anspruch 7, worin die Abkühlphase der Temperaturoszillationsbehandlung bei einer Temperatur zwischen etwa -5°C und 20°C durchgeführt wird.

18. Verfahren nach Anspruch 7, worin die für die Abkühlphase der Temperaturoszillationsbehandlung ausgewählte Temperatur niedriger liegt als die für die entsprechende Aufheizphase der Temperaturoszillationsbehandlung ausgewählte Temperatur.

19. Verfahren nach Anspruch 7, worin die Kristallsuspension mit Irbesartan-Kristallen angeimpft wird, deren Verhältnis zwischen Länge und Breite zwischen 1:1 und 10:1 liegt.

20. Verfahren zur Herstellung einer Verbindung nach einem der Ansprüche 1 bis 3, **dadurch gekennzeichnet, daß** es die Stufen umfaßt, die in folgendem bestehen:
a) Herstellung einer Lösung von Irbesartan der Form A mit Nadelhabitus in einem Alkohol unter solchen Konzentrations- und Temperaturbedingungen, die eine vollständige Lösung des Irbesartans ermöglichen;
b) Abkühlen der Lösung auf eine Temperatur, die in Abhängigkeit von der Konzentration der Lösung in der Weise ausgewählt wird, daß die Lösung im metastabilen Bereich liegt;
c) Animpfen mit Irbesartankristallen mit Ziegelhabitus;
d) Abkühlen der Irbesartanlösung auf eine Temperatur zwischen etwa 20°C und 5°C;
e) Unterwerfen der in dieser Weise gebildeten Kristallsuspension einer mechanischen Scherbehandlung mit Hilfe einer Schervorrichtung;
f) Wiedererhitzen der Kristallsuspension auf eine Temperatur zwischen etwa 40°C und 60°C zum Auflösen der feinen Teilchen;
g) Abkühlen der Kristallsuspension auf eine Temperatur zwischen etwa 20°C und 5°C;
h) Abfiltrieren der in dieser Weise gebildeten Kristalle mit Ziegelhabitus.

21. Verfahren nach Anspruch 20, worin in der Stufe a) Irbesartan in Lösung in Isopropanol vorliegt.

22. Verfahren nach Anspruch 20, worin in der Stufe b) eine Lösung, die 50 g/Liter bis 70 g/Liter Irbesartan in Isopropanol enthält, auf eine Temperatur zwischen 60°C und 80°C abgekühlt wird.

23. Verfahren nach Anspruch 20, worin in der Stufe c) die Lösung mit Irbesartankristallen angeimpft wird, deren Verhältnis zwischen Länge und Breite zwischen 1:1 und 10:1 liegt.

24. Verfahren nach Anspruch 23, worin die angeimpfte Lösung vor dem Abkühlen während einiger Minuten bis etwa 2 Stunden bei einer Temperatur zwischen 80°C und 22°C gehalten wird.

25. Verfahren nach Anspruch 21, worin in den Stufen b) und d) die Abkühlgeschwindigkeit etwa 5°C bis 20°C pro Stunde beträgt.

26. Verfahren nach Anspruch 20, worin in der Stufe e) die mechanische Scherbehandlung mit einer Vorrichtung durchgeführt wird, die eine Rotationsgeschwindigkeit von etwa 10 000 min⁻¹ bis 15 000 min⁻¹ aufweist.

27. Verfahren nach Anspruch 26, worin die mechanische Scherbehandlung der Stufe e) entweder in der Weise erfolgt, daß man die Vorrichtung zur Scherbehandlung direkt in das Reaktionsgefäß einbringt oder man die Kristallsuspension durch die Vorrichtung zur Scherbehandlung führt.

28. Pharmazeutische Zubereitung, enthaltend eine Verbindung nach einem der Ansprüche 1 bis 3 und geeignete pharmazeutische Trägermaterialien.

29. Pharmazeutische Zubereitung nach Anspruch 28, enthaltend ein Diuretikum in Kombination mit einer Verbindung nach einem der Ansprüche 1 bis 3.

30. Pharmazeutische Zubereitung nach Anspruch 29, worin das Diuretikum Hydrochlorothiazid ist.

## Claims

1. Crystalline compound of formula: 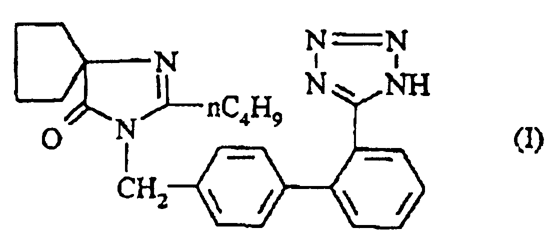 having a crystal habit such that the ratio between the length and the width of the crystals is between 1:1 and 10:1 and the chargeability of which, measured by tribogeneration, ranges between 0 and -10 nanocoulombs/g.

2. Crystalline compound according to Claim 1, in which the ratio between the length and the width of the crystals is between 1:1 and 5:1.

3. Novel crystalline form of irbesartan of form A, **characterized in that** the ratio between the length and the width of the crystals is between 1:1 and 5:1 and the chargeability, of which, measured by tribogeneration, ranges between 0 and -10 nanocoulombs/g.

4. Process for preparing a compound according to any one of Claims 1 to 3, **characterized in that** a crystalline suspension of a compound of formula (I) is subjected to at least one sonication episode and at least one temperature oscillation episode.

5. Process for preparing a compound according to any one of Claims 1 to 3, **characterized in that** a crystalline suspension of irbesartan of acicular habit form A is subjected to at least one sonication episode and at least one temperature oscillation episode.

6. Process according to either of Claims 4 and 5, in which the temperature oscillation episode comprises a heating phase and a corresponding cooling phase.

7. Process according to Claim 6, in which the heating phase precedes the cooling phase.

8. Process according to Claim 7, in which the sonication episode is followed by a temperature oscillation episode.

9. Process according to either of Claims 4 and 5, in which the sonication episode is preceded by a temperature oscillation episode.

10. Process according to either of Claims 4 and 5, in which the sonication episode is carried out simultaneously with the temperature oscillation episode.

11. Process according to either of Claims 4 and 5, in which a sonication episode is carried out between 2 temperature oscillation episodes.

12. Process according to either of Claims 4 and 5, in which the sonication and/or temperature oscillation episodes are repeated independently.

13. Process according to either of Claims 4 and 5, in which the sonication is carried out in batches, semi-continuously or continuously.

14. Process according to Claim 7, in which the heating phase of the temperature oscillation episode is carried out at a temperature of between about 20°C and 100°C.

15. Process according to Claim 7, in which the heating phase of the temperature oscillation episode is carried out at a temperature such that about 15% to 25% of the crystals are dissolved in about 60 minutes.

16. Process according to Claim 7, in which the cooling phase of the temperature oscillation episode is carried out at a temperature of between about 100°C and -20°C.

17. Process according to Claim 7, in which the cooling phase of the temperature oscillation episode is carried out at a temperature of between about -5°C and 20°C.

18. Process according to Claim 7, in which the temperature selected for the cooling phase of the temperature oscillation episode is less than the temperature selected for the corresponding heating phase of the temperature oscillation episode.

19. Process according to Claim 7, in which the crystalline suspension is seeded with irbesartan crystals whose ratio between the length and the width is between 1:1 and 10:1.

20. Process for preparing a compound according to any one of Claims 1 to 3, **characterized in that** it contains the steps consisting in:
a) preparing a solution of irbesartan acicular habit form A in an alcohol, under concentration and temperature conditions which allow the total solubility of the irbesartan;
b) cooling the said solution to a temperature selected as a function of the concentration of the solution, such that the solution is in the metastable zone;
c) seeding with irbesartan crystals of brick habit;
d) cooling the irbesartan solution to a temperature of between about 20°C and 5°C;
e) subjecting the crystalline suspension thus formed to a mechanical shearing using a shearing machine;
f) heating the crystalline suspension to a temperature of between about 40°C and 60°C to dissolve the fine particles;
g) cooling the crystalline suspension to a temperature of between about 20°C and 5°C;
h) filtering off the crystals of brick habit thus formed.

21. Process according to Claim 20, in which, in step a), the irbesartan is dissolved in isopropanol.

22. Process according to Claim 20, in which, in step b), a solution containing 50 g/litre to 70 g/litre of irbesartan in isopropanol is cooled to a temperature ranging between 60°C and 80°C,

23. Process according to Claim 20, in which, in step c), the solution is seeded with irbesartan crystals whose ratio between the length and the width is between 1:1 and 10:1.

24. Process according to Claim 23, in which the seeded solution is maintained at a temperature of between 80°C and 22°C for a few minutes to about 2 hours, before being cooled.

25. Process according to Claim 21, in which, in steps b) and d), the rate of cooling is from about 5°C to 20°C per hour.

26. Process according to Claim 20, in which, in step e), the mechanical shearing is carried out by a machine having a spin speed of about 10 000 rpm to 15 000 rpm.

27. Process according to Claim 26, in which the mechanical shearing in step e) is carried out either by placing the shearing machine directly in the reactor or by passing the crystalline suspension into the shearing machine.

28. Pharmaceutical composition containing a compound according to any one of Claims 1 to 3 and pharmaceutically acceptable excipients.

29. Pharmaceutical composition according to Claim 28, containing a diuretic agent combined with a compound according to any one of Claims 1 to 3.

30. Pharmaceutical composition according to Claim 29, in which the diuretic agent is hydrochlorothiazide.
